# EUROPEAN PATENT APPLICATION

(11) **EP 4 582 148 A1**
(43) Date of publication of application: **09.07.2025**
(21) Application number: 23859930.2
(22) Date of filing: 31.07.2023
(51) Int. Cl.: A61P 39/06, A61P 43/00, A61P 17/18, A61Q 19/00, A61K 36/33, A61K 8/9789

(54) **ANTIOXIDANT AND FIBROBLAST PROMOTER**

(30) Priority: 29.08.2022 JP 2022135848
(71) Applicant: Asfreya Inc., Tokyo 136-0082 (JP)
(72) Inventor: OCHIYA Takahiro, Tokyo 104-0053 (JP)
(74) Representative: J A Kemp LLP
(86) International application number: PCT/JP2023/027948
(87) International publication number: WO 2024/048161

(57) **Abstract**

An object of the present invention is to provide a novel ingredient for improving various conditions in living organisms, such as sagging skin. An antioxidant of the present invention is characterized by containing extracellular vesicles derived from a dragon fruit. A fibroblast promoter of the present invention is characterized by containing extracellular vesicles derived from a dragon fruit. The dragon fruit is, for example, a red-fleshed dragon fruit.

## Description

### Technical Field

The present invention relates to an antioxidant, a fibroblast promoter, and uses thereof.

### Background Art

Various ingredients are being developed to improve conditions in living organisms, including the skin. In particular, active ingredients derived from natural resources are in demand in light of their safety and the like.

### Summary of the Invention

### Technical Problem

Therefore, an object of the present invention is to provide a novel ingredient for improving various conditions in living organisms, such as sagging skin.

### Solution to the Problem

To achieve the above object, an antioxidant of the present invention is characterized by containing extracellular vesicles derived from a dragon fruit.

A fibroblast promoter of the present invention is characterized by containing extracellular vesicles derived from a dragon fruit.

### Advantageous Effects of Invention

The inventor of the present invention newly discovered that extracellular vesicles derived from a dragon fruit have antioxidant capacity and can also promote the expression of genes involved in the production of collagen and elastin in fibroblasts that produce these proteins. Thus, according to the present invention, for example, oxidation can be prevented, and the production of collagen, elastin, and the like can be promoted, and therefore, it is possible to improve conditions in living organisms associated with these proteins.

### Brief Description of Drawings

[FIG. 1] FIG. 1 is a graph showing a particle size distribution of extracellular vesicles derived from a red-fleshed dragon fruit in Example 1.
[FIG. 2] FIG. 2 is a graph showing the antioxidant capacity of extracellular vesicles derived from a red-fleshed dragon fruit in Example 2.
[FIG. 3] FIG. 3 is a graph showing the effect of extracellular vesicles derived from a red-fleshed dragon fruit on fibroblasts in Example 3.
[FIG. 4] FIG. 4 is a graph showing a particle size distribution of extracellular vesicles derived from a red-fleshed dragon fruit in Example 4.
[FIG. 5] FIG. 5 shows the results of Western blotting detecting a marker in an EV sample derived from a red-fleshed dragon fruit in Example 5.

### Description of Embodiments

[1] An antioxidant containing extracellular vesicles derived from a dragon fruit.
[2] The antioxidant according to clause [1], wherein the extracellular vesicles are at least either one of extracellular vesicles derived from dragon fruit juice and extracellular vesicles derived from dragon fruit leaves.
[3] The antioxidant according to clause [1] or [2], wherein a particle size distribution of the extracellular vesicles has a mean of 50 to 500 nm.
[4] The antioxidant according to any one of clauses [1] to [3], which is for parenteral use.
[5] The antioxidant according to clause [4], which is for transdermal use.
[6] The antioxidant according to any one of clauses [1] to [3], which is for oral use.
[7] The antioxidant according to any one of clauses [1] to [6], wherein the dragon fruit is a red-fleshed dragon fruit.
[8] A fibroblast promoter containing extracellular vesicles derived from a dragon fruit.
[9] The fibroblast promoter according to clause [8], wherein the extracellular vesicles are at least either one of extracellular vesicles derived from dragon fruit juice and extracellular vesicles derived from dragon fruit leaves.
[10] The fibroblast promoter according to clause [9], wherein a particle size distribution of the extracellular vesicles has a mean of 50 to 500 nm.
[11] The fibroblast promoter according to any one of clauses [8] to [10], which is for parenteral use.
[12] The fibroblast promoter according to clause [11], which is for transdermal use.
[13] The fibroblast promoter according to any one of clauses [8] to [10], which is for oral use.
[14] The fibroblast promoter according to any one of clauses [8] to [13], which promotes at least one of collagen production and elastin production in fibroblasts.
[15] The fibroblast promoter according to any one of clauses [8] to [14], wherein the dragon fruit is a red-fleshed dragon fruit.
[16] An antioxidation method including adding extracellular vesicles derived from a dragon fruit to a subject.
[17] A method for promoting collagen production in fibroblasts, the method including bringing extracellular vesicles derived from a dragon fruit into contact with fibroblasts.
[18] A method for promoting elastin production in fibroblasts, the method including bringing extracellular vesicles derived from a dragon fruit into contact with fibroblasts.
[19] A method for promoting elastin production in fibroblasts, the method including bringing extracellular vesicles derived from a dragon fruit into contact.

Unless otherwise stated, terms used in this specification can be used in the sense normally used in the art.

In this specification, "extracellular vesicles" are membrane vesicles secreted from cells and are hereinafter also referred to as EVs. Examples of the extracellular vesicles include exosomes derived from endosomes secreted by an endocytosis pathway, microvesicles derived from plasma membranes, and the like.

### (1) Extracellular Vesicles Derived from Dragon Fruit

As described above, the antioxidant of the present invention and the fibroblast promoter of the present invention are characterized by containing extracellular vesicles derived from a dragon fruit. The extracellular vesicles derived from a dragon fruit will be described first below.

In the present invention, as the extracellular vesicles, any extracellular vesicles can be used as long as they are derived from a dragon fruit. Dragon fruit refers to plants of the genus Hylocereus in the family Cactaceae. In the present invention, dragon fruit may be the fruit, the whole plant body including the fruit, or each part, and can include any of these meanings, unless otherwise indicated. Dragon fruit is also called pitaya. The type of the dragon fruit is not particularly limited. For example, red-fleshed dragon fruit with red flesh (*Hylocereus costaricensis, Hylocereus polyrhizus*)*,* white-fleshed dragon fruit with white flesh (*Hylocereus undatus*), golden dragon fruit with yellow skin and white flesh (*Hylocereus polyrhizus*), pink dragon fruit with pink flesh (hybrid: *Hylocereus undatus*× *Hylocereus ocanponis*), and the like may be used, of which red-fleshed dragon fruit is preferable.

To prepare the extracellular vesicles, for example, the entire plant body of a dragon fruit or any part of a dragon fruit may be used as a dragon fruit material. The part above may be, for example, fruit, fruit skin, fruit flesh, seeds, fruit flesh with seeds, leaves, or the like, or alternatively, a combination of any two or more parts, or the like may be used. Preferably, fruit flesh or fruit flesh with seeds can be used as the material above. The fruit may be, for example, fully ripe or unripe, but is preferably fully ripe.

The extracellular vesicles derived from a dragon fruit can be prepared from, for example, a liquid fraction derived from the dragon fruit material. The liquid fraction may be, for example, a squeezed liquid obtained by squeezing the dragon fruit material, a mixed liquid of a crushed product of the dragon fruit material and a solvent, or an extracted liquid obtained from the dragon fruit material. The squeezing method is not particularly limited, and, for example, pressing, rotating, rotary pressing, and the like may be used. Specifically, for example, a squeezed liquid (juice) obtained by squeezing the fruit, fruit flesh, or fruit flesh with seeds, which is used as the dragon fruit material, is preferable. For example, the squeezed liquid above is preferably a squeezed liquid from the fruit flesh or a squeezed liquid from the fruit flesh with seeds, and more preferably a squeezed liquid from the fruit flesh from which seeds have been removed. A squeezed liquid from the fruit flesh from which seeds have been removed can be prepared by, for example, removing seeds from a squeezed liquid obtained by squeezing the fruit flesh with seeds, and the removal of the seeds can be performed by centrifuging the squeezed liquid from the fruit flesh with seeds and removing the sediment containing the seeds. The extraction method is not particularly limited, and, for example, a method may be used in which a solvent is added to the crushed product and a liquid fraction is collected as an extracted liquid. The liquid fraction may be, for example, a concentrated liquid of the squeezed liquid, the mixed liquid, the extracted liquid, or the like. The crushed product of the dragon fruit material may be, for example, in paste form. For example, a crushed product of the fruit flesh with seeds may be used as the crushed product above. The solvent above is not particularly limited, and may be, for example, an aqueous solvent, such as water, a buffer solution such as a phosphate buffer (PBS), or physiological saline.

The dragon fruit material and the crushed product, for example, may be used immediately after preparation or may be used after storage. When used after storage, for example, they may be used after storage at room temperature, after refrigerated storage, or after frozen storage, or after being returned to room temperature after refrigerated storage or frozen storage. The liquid fraction may be, for example, a freshly prepared liquid, a liquid after storage at room temperature, a liquid after refrigerated storage, or a liquid after frozen storage.

As the liquid fraction, for example, the squeezed liquid, the mixed liquid, or the extracted liquid may be used as it is, or a concentrated liquid thereof may be used. The concentrated liquid may be, for example, a concentrated liquid obtained by concentrating the squeezed liquid, the mixed liquid, or the extracted liquid. A concentrate of the squeezed liquid is also referred to as a dragon fruit extract, for example. In addition, as the liquid fraction, for example, a mixed liquid (also referred to as a concentrated and reconstituted liquid) of a dried product of the squeezed liquid, or a dried product of the extracted liquid, with a solvent may also be used. The dried product can be prepared by, for example, drying the squeezed liquid or the extracted liquid, and is preferably in powder form for easier handling, for example. The drying process may be, for example, lyophilization or the like.

The preparation of extracellular vesicles from the liquid fraction can be performed, for example, under cooling conditions or at room temperature (e.g., 4°C to 37°C), and centrifugation and the like are preferably performed at 4°C ± 2°C, for example.

Contaminants such as fibers and dust may be removed from the liquid fraction prior to the preparation of extracellular vesicles, for example. The removal of contaminants may be performed through, for example, separation by standing, centrifugation, or filtration. In the case of standing, for example, the liquid fraction is stirred, allowed to stand, and the supernatant is then collected. Thus, precipitated contaminants can be removed. In the case of centrifugation, the liquid fraction is subjected to coarse centrifugation, and the supernatant is collected. Thus, contaminants can be removed. For example, the liquid fraction may be subjected to contaminant removal through standing and subsequently subjected to further contaminant removal through the aforementioned coarse centrifugation. The conditions for the standing are not particularly limited and the standing may be performed, for example, for 5 to 60 minutes (e.g., 20 minutes). The conditions for the coarse centrifugation are not particularly limited and the coarse centrifugation may be performed, for example, at 400 to 3000 ×g (e.g., 2000 ×g) for 5 to 30 minutes (e.g., 10 minutes). It is preferable to confirm the presence of residual contaminants in the liquid fraction that has been subjected to the contaminant removal process by, for example, performing particle observation using a nanoparticle analysis system or the like.

The aforementioned filtration may be, for example, filtration using a filter medium, or the like. In the case of removing contaminants from the liquid fraction using the filter medium, for example, residues on the filter medium are removed as contaminants, and the filtrate fraction is collected. For example, a filter or the like can be used as the filter medium. The pore size of the filter medium is not particularly limited, and, for example, any pores that allow the extracellular vesicles to pass through can be employed. The liquid fraction after the standing or the coarse centrifugation may be further subjected to filtration using a filter medium.

The method for preparing the extracellular vesicles from the liquid fraction is not particularly limited, and, for example, ultracentrifugation can be employed. Specifically, ultracentrifugation can be performed on the liquid fraction to collect the precipitated fraction containing extracellular vesicles, as an extracellular vesicle fraction. The conditions for the ultracentrifugation are not particularly limited and the ultracentrifugation may be performed, for example, at 50,000 to 150,000 ×g (e.g., 100,000 ×g) for 50 to 140 minutes (e.g., 70 minutes). The precipitated fraction may, for example, be suspended in the solvent and stored refrigerated or stored frozen.

As a specific example, when using the squeezed liquid (juice), for example, the juice is stirred, allowed to stand at a temperature between 4°C and room temperature, and a liquid is collected so as to remove precipitated contaminants. Then, the coarse centrifugation is performed on the collected liquid, the supernatant fraction is collected, ultracentrifugation is further performed on the supernatant fraction, and the precipitated fraction is collected as the extracellular vesicle fraction.

When using a dried product (e.g., a powder) of the squeezed liquid, for example, the dried product is suspended in a solvent, contaminants are removed through the coarse centrifugation, and the ultracentrifugation is performed on the collected liquid fraction. Thus, the extracellular vesicle fraction can be obtained.

When using an extract obtained by concentrating the squeezed liquid, for example, the solvent is added to the extract and the mixture is stirred, as necessary, the coarse centrifugation is performed, the supernatant fraction is collected, ultracentrifugation is further performed on the supernatant fraction, and the precipitated fraction is collected as the extracellular vesicle fraction.

Additionally, for example, filtration (also referred to as ultrafiltration) using a filter medium may also be employed as the method for preparing the extracellular vesicles from the liquid fraction. In this case, for example, a filter medium with pores of a size that allows dragon fruit extracellular vesicles, which will be described later, to pass through, a filter medium with pores of a size that does not allow the extracellular vesicles to pass through, or a combination of both can be used.

The particle size distribution of the extracellular vesicles has a mean of, for example, 50 to 500 nm.

The extracellular vesicles used in the present invention may have a mean of, for example, 320 ± 120 nm, 320 ± 100 nm, 320 ± 80 nm, 320 ± 50 nm, 320 ± 30 nm, 320 ± 20 nm, 320 ± 10 nm, or 320 ± 5 nm. When the mean of the extracellular vesicles is 320 ± 120 nm, the mode of the particle size distribution is, for example, 290 ± 90 nm, 290 ± 87 nm, 290 ± 80 nm, 290 ± 50 nm, 290 ± 30 nm, 290 ± 20 nm, 290 ± 10 nm, or 290 ± 5 nm. The mode can also be referred to as, for example, the peak of the particle size distribution. Furthermore, in this case, the SD (standard deviation) of the particle size distribution of the extracellular vesicles is, for example, 80 ± 40 nm, 80 ± 30 nm, 80 ± 20 nm, 80 ± 10 nm, or 80 ± 5 nm.

On the other hand, the extracellular vesicles used in the present invention may also have a mean of, for example, 83 ± 21 nm, 83 ± 30 nm, 83 ± 20 nm, 83 ± 10 nm, 83 ± 5 nm, 83 ± 30 nm, 83 ± 20 nm, 83 ± 10 nm, or 83 ± 5 nm. When the mean of the extracellular vesicles is 83 ± 21 nm, the mode of the particle size distribution is, for example, 67 ± 20 nm, 67 ± 10 nm, or 67 ± 5 nm.

The method for measuring the particle size distribution of the extracellular vesicles is not particularly limited. For example, light scattering methods, such as a laser diffraction method and a dynamic light scattering method, a particle tracking analysis method, a nano-tracking analysis method, and the like can be used. For example, a commercially available device, such as NanoSight (trade name, Quantum Design), can be used for the measurement.

### (2) Antioxidant

As described above, the antioxidant of the present invention is characterized by containing extracellular vesicles derived from a dragon fruit. The extracellular vesicles contained in the antioxidant are, for example, isolated from the dragon fruit. The antioxidant of the present invention is characterized by containing the extracellular vesicles, and other configurations and conditions are not particularly limited. With respect to the extracellular vesicles derived from a dragon fruit, the descriptions in (1) above can be referred to.

The antioxidant of the present invention may, for example, be composed exclusively of the extracellular vesicles derived from a dragon fruit, or it may also contain an additional component. The additional component may be, for example, another component in the liquid fraction derived from the dragon fruit, or even another additive component. The other additive component is not particularly limited and can be appropriately selected based on the target site where the antioxidant of the present invention is to be used, the method of use, and the like.

The antioxidant of the present invention may be, for example, for pharmaceutical uses, specified health uses, or cosmetic uses. The antioxidant of the present invention may be, for example, a composition containing the extracellular vesicles derived from a dragon fruit.

When the antioxidant of the present invention is a pharmaceutical composition, the route of administration thereof is not particularly limited and may be, for example, parenteral or oral. In the case of the parenteral administration, examples of administration routes include transdermal, subcutaneous, intravenous, intra-arterial, intraperitoneal, intranasal, topical, enteral, and the like. When the antioxidant of the present invention is a pharmaceutical composition, its form is not particularly limited and can be appropriately selected based on the route of administration. Specific examples of the form include, for example, liquid formulations, such as solutions and suspensions, emulsions, gels, sols, ointments, granules, tablets, capsules, and the like. When the antioxidant of the present invention is for oral use, the composition may be, for example, a so-called supplement.

When the antioxidant of the present invention is a cosmetic composition, the route of administration thereof is, for example, transdermal, and a specific application site may be, for example, the skin of the face, the neck, the upper half of the body, the lower half of the body, or the like. When the antioxidant of the present invention is a cosmetic composition, its form is not particularly limited. Examples of the form include lotions, gels, milky lotions, creams, oils, ointments, and the like.

The other additive component is not particularly limited and can be appropriately selected based on the route of administration and the form. Specific examples include, for example, excipients, diluents, oxidation inhibitors, preservatives, extenders, wetting agents, thickeners, viscosity stabilizers, UV shielding agents, binders, vitamins, perfumes, coloring matter, and the like. The other additive component may be, for example, a pharmaceutically acceptable component or the like.

The content of the extracellular vesicles derived from a dragon fruit in the antioxidant of the present invention is not particularly limited and can be appropriately set based on, for example, the type, age, sex, conditions, and the like of the living organism to which the antioxidant is to be administered. As a specific example, in the case of a transdermal composition, the content of the extracellular vesicles may be, for example, 0.01% to 5% (w/w). In the case of an oral composition, the daily dosage of the extracellular vesicles is, for example, 5 to 1000 mg, 25 mg, or the like, and the daily dosing frequency is not particularly limited but may be, for example, once to three times, once to twice, or once.

The antioxidant of the present invention can be administered to, for example, humans or non-human animals. Examples of the non-human animals include mice, rats, rabbits, pigs, cattle, camels, dogs, cats, and the like.

### (3) Fibroblast Promoter

The fibroblast promoter of the present invention is characterized by containing the extracellular vesicles derived from a dragon fruit. The fibroblast promoter of the present invention is characterized by containing the extracellular vesicles, and other configurations and conditions are not particularly limited. With respect to the extracellular vesicles derived from a dragon fruit, the descriptions in (1) above can be referred to. Also, with respect to the fibroblast promoter of the present invention, the descriptions above regarding the antioxidant of the present invention can be referred to. In the present invention, the promotion of fibroblasts means, for example, the promotion of elastin production or the promotion of collagen production.

### (4) Improvement of Conditions

As described above, the extracellular vesicles derived from a dragon fruit exhibit, for example, antioxidant capacity and fibroblast-promoting ability. The fibroblast-promoting ability refers to, for example, the promotion of collagen and/or elastin production by the fibroblasts. Therefore, the antioxidant of the present invention and the fibroblast promoter of the present invention can improve skin conditions. For example, they can improve wrinkles, improve sagging skin, prevent aging, moisturize the skin, reduce/remove nasolabial folds, improve skin quality, tighten open pores, improve sagging pores, cleanse pores, remove keratin plugs, improve acne scars, exfoliate, remove dark spots, improve skin texture, increase skin clarity, improve barrier function, normalize stratum corneum turnover, increase beneficial skin bacteria, and so on. In the present invention, "improve" means, for example, prevention of worsening, alleviation of conditions, healing of conditions, and the like. The antioxidant of the present invention and the fibroblast promoter of the present invention can also be referred to as compositions for improving conditions, for example.

### (5) Antioxidation Method

The antioxidation method of the present invention is characterized by including administering the antioxidant of the present invention. The present invention is characterized by the use of the antioxidant of the present invention, and other steps and conditions are not limited in any way. With respect to the present invention, the descriptions above regarding the antioxidant of the present invention can be referred to.

The method of administration used for the administering is not particularly limited, and as described above, oral administration or parenteral administration may be used. In the case of the parenteral administration, examples of administration routes include transdermal, subcutaneous, intravenous, intra-arterial, intraperitoneal, intranasal, topical, enteral, and the like. In the case of transdermal administration, specific examples of the application site include the skin of the face, the neck, the upper half of the body, the lower half of the body, or the like.

The form of administration is not particularly limited and may be, for example, *in vivo* or *in vitro.* In the case of *in vitro* administration, the administration target (also referred to as a subject) may be, for example, a cell, a tissue, an organ, or the like. In the case of *in vivo* administration, the administration target may be, for example, a human, or a non-human animal as described above. Also, in the case of *in vivo* administration, for example, the administration may be performed either orally or parenterally.

The conditions for administration are not particularly limited and can be appropriately set based on, for example, the method of administration, the site of administration, the type of the administration target, and the age, sex, conditions, and the like of the administration target.

### (6) Method for Promoting Fibroblasts

The method for promoting fibroblasts according to the present invention is characterized by including administering the fibroblast promoter of the present invention. The present invention is characterized by the use of the fibroblast promoter of the present invention, and other steps and conditions are not limited in any way. With respect to the present invention, the descriptions above regarding the fibroblast promoter of the present invention can be referred to. The method for promoting fibroblasts according to the present invention means, for example, the promotion of elastin production or collagen production in fibroblasts.

The method of administration used for the administering is not particularly limited, and as described above, oral administration or parenteral administration may be used. In the case of the parenteral administration, examples of administration routes include transdermal, subcutaneous, intravenous, intra-arterial, intraperitoneal, intranasal, topical, enteral, and the like. In the case of transdermal administration, specific examples of the application site include the skin of the face, the neck, the upper half of the body, the lower half of the body, or the like.

The form of administration is not particularly limited and may be, for example, *in vivo* or *in vitro.* In the case of *in vitro* administration, the administration target may be, for example, a cell, a tissue, an organ, or the like. In the case of *in vivo* administration, the administration target may be, for example, a human, or a non-human animal as described above. Also, in the case of *in vivo* administration, for example, the administration may be performed either orally or parenterally.

The conditions for administration are not particularly limited and can be appropriately set based on, for example, the method of administration, the site of administration, the type of the administration target, and the age, sex, conditions, and the like of the administration target.

### (7) Uses

The present invention relates to extracellular vesicles derived from a dragon fruit for antioxidative use. The present invention relates to extracellular vesicles derived from a dragon fruit for use in the production of an antioxidant.

The present invention relates to extracellular vesicles derived from a dragon fruit for use in fibroblast promotion. The present invention relates to extracellular vesicles derived from a dragon fruit for use in the production of a fibroblast promoter.

With respect to the extracellular vesicles derived from a dragon fruit of the present invention, the descriptions above can be referred to.

Hereinafter, the present invention will be described in detail by means of examples and the like, but the present invention is not limited to these examples and the like.

### Examples

### [Example 1]

Extracellular vesicles were collected from a red-fleshed dragon fruit material.

### (1) Powder

First, 1 g of a commercially available powder (trade name: Red Dragon Fruit Powder, Bio Actives Japan Corporation) made from the juice of a red-fleshed dragon fruit (a squeezed liquid obtained by squeezing the fruit flesh after removing seeds) was mixed with 1 ml of sterile distilled water. The resulting mixed liquid was stirred thoroughly and allowed to stand at room temperature for 20 minutes. Then, precipitated contaminants were removed, and the supernatant was collected. The supernatant was filtered through a filter with a pore size of 0.22 µm, and the filtrate was collected. The filtrate was then subjected to ultracentrifugation (100,000 ×g, 70 minutes, 4°C), and the precipitated fraction (pellet) was collected. Subsequently, 1 g of the pellet was suspended in 10 ml of distilled water and collected as an EV sample. The EV sample was analyzed using a nanoparticle analysis system (trade name: NanoSight, LM10, laser 405 nm, Malvern Panalytical), and the particle size distribution of extracellular vesicles contained in the EV sample was confirmed. The analysis software used was NTA3.4 (Malvern Panalytical).

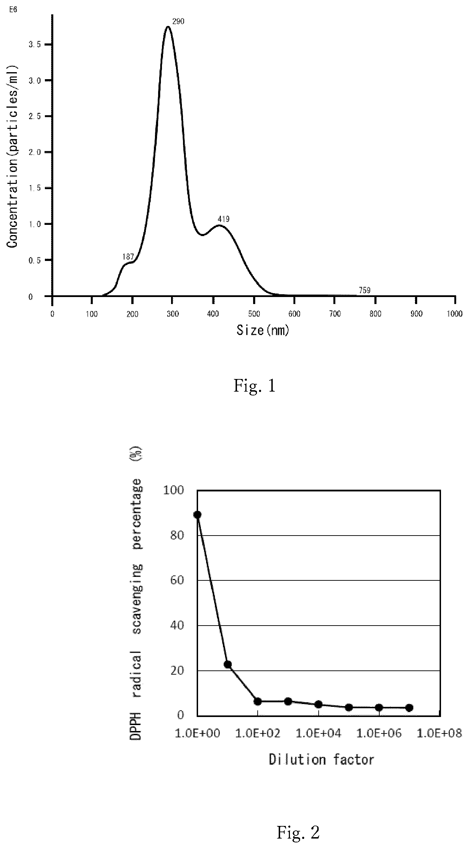

The results of the particle size distribution are shown in the graph of FIG. 1. In FIG. 1, the vertical axis indicates the particle concentration (particles/ml), and the horizontal axis indicates the particle size (nm). The EV sample (1 g pellet/10 ml) had an extracellular vesicle concentration of 8.92 × 10¹⁰ particles/ml, and the extracellular vesicle concentration of a further 200-fold diluted sample was calculated to be 4.46 × 10⁸ particles/ml.
Mean: 319.9 nm
Mode: 589.6 nm
SD: 80.8 nm

### [Example 2]

Extracellular vesicles derived from a red-fleshed dragon fruit were evaluated for antioxidant capacity.

The EV sample prepared in Example 1 above was diluted with distilled water to prepare a diluted sample 1 (8 × 10¹⁰ particles/ml). The diluted sample 1 was further diluted with distilled water to prepare a plurality of diluted samples. These diluted samples were then evaluated for antioxidant capacity using a commercially available kit (trade name: DPPH Antioxidant Assay Kit, Dojindo, D678) according to its instructions. With this kit, the ability to scavenge artificially generated radicals (2,2-diphenyl-1-picrylhydrazyl: DPPH) can be evaluated as antioxidant capacity.

The results are shown in the graph of FIG. 2. In FIG. 2, the vertical axis indicates the DPPH radical scavenging percentage (%), and the horizontal axis indicates the dilution factor of the EV sample. As shown in FIG. 2, it was found that the use of the diluted sample 1 (1.0E+00), which had an extracellular vesicle concentration of 8 × 10¹⁰ particles/ml, effectively scavenged 90% of the radicals.

### [Example 3]

The effect of extracellular vesicles derived from a red-fleshed dragon fruit on fibroblasts was evaluated.

The diluted sample 1 (8 × 10¹⁰ particles/ml) of EVs prepared in Example 1 above was used as a sample. The expression levels of a target gene (Collagen I (COL1A1) Human) involved in type I collagen production and a target gene (Elastin (ELN) Human) involved in elastin production in human fibroblasts (Human Dermal Fibroblast, Normal, Cryopreserved <NHDF-c> (Funakoshi)) were measured using the following method (n = 2). The actin gene expression level was also measured as a control for expression levels. Cells were cultured using a dedicated culture medium (Fibroblast Basal Medium, Funakoshi), and in a subconfluent state, the EVs were added. The amount of EVs added was set to a concentration where 100 EV particles were taken up per cell. Cells were collected 72 hours after the addition, and mRNA was purified using an RNAeasy reagent (QIAGEN). On the other hand, as a reference example, the expression levels of the aforementioned genes were measured (n=2) in the same manner using exosomes (mean of particle size distribution: 110 nm) derived from adipose-derived mesenchymal stem cells (ADMSCs), which are known to promote collagen production and elastin production in fibroblasts.

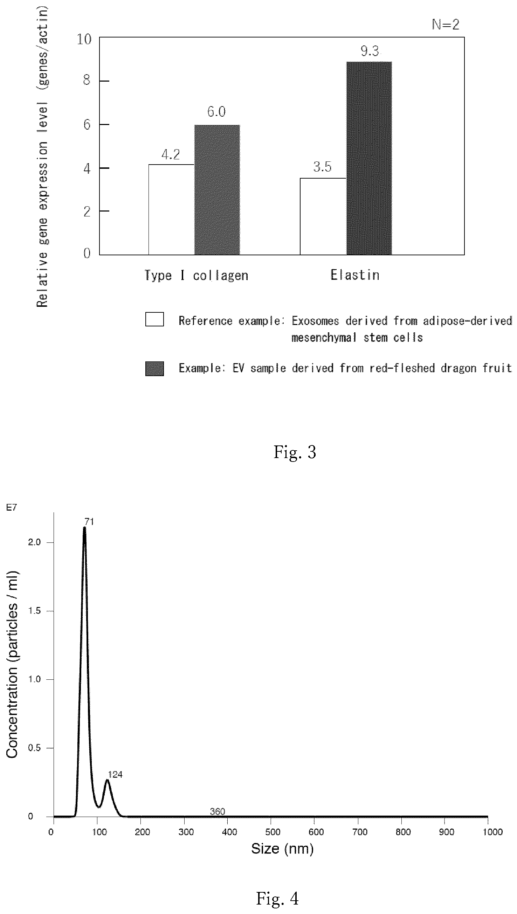

The results are shown in the graph of FIG. 3. In FIG. 3, the vertical axis indicates the relative gene expression level, specifically expressed as "target gene expression level / actin gene expression level". As shown in FIG. 3, the EV sample derived from the red-fleshed dragon fruit was found to be able to promote the expression of both the target gene involved in type I collagen production and the target gene involved in elastin production even more compared to the exosomes derived from ADMSCs in the reference example.

### [Example 4]

Extracellular vesicles were collected from a red-fleshed dragon fruit material.

### (1) Extract

First, 1 g of a commercially available extract (manufactured by Shirakawa Farm) made from the juice of a red-fleshed dragon fruit (a squeezed liquid obtained by squeezing the fruit flesh after removing seeds) was dissolved in 1 ml of sterile distilled water. The resulting solution was subjected to centrifugation (2,000 ×g, 10 minutes, 4°C), and the supernatant was collected. The supernatant was further subjected to ultracentrifugation (100,000 ×g, 70 minutes, 4°C), and the precipitated fraction (pellet) was collected. Subsequently, 1 g of the pellet was suspended in 10 ml of distilled water and collected as an EV sample. The EV sample was analyzed using a nanoparticle analysis system (trade name: NanoSight, LM10, laser 405 nm, Malvern Panalytical), and the particle size distribution of extracellular vesicles contained in the EV sample was confirmed. The analysis software used was NTA3.4 (Malvern Panalytical).

The results of the particle size distribution are shown in the graph of FIG. 4. In FIG. 4, the vertical axis indicates the particle concentration (particles/ml), and the horizontal axis indicates the particle size (nm). The EV sample (1 g pellet/10 ml) had an extracellular vesicle concentration of 2 × 10¹⁰ particles/ml.
Mean: 82.9 nm
Mode: 67.1 nm

### [Example 5]

An EV sample collected from a red-fleshed dragon fruit material was subjected to detection of a plant-derived extracellular vesicle marker.

HSC70 (molecular weight: 70 kDa) is generally known as a marker for plant-derived extracellular vesicles. Therefore, in the EV sample isolated by the ultracentrifugation method described in Example 4, HSC70 was detected by Western blotting under reducing conditions using an anti-HSC70 antibody. As the antibody, an antibody (Anti-HSC70 (Plant) Antibody, Catalog# SPC-302D, StressMarq Biosciences Inc.) against plant HSC70 was used. In addition, as a positive control for plant-derived extracellular vesicles, extracellular vesicles derived from spinach leaves, which are known to be HSC70 positive, were used. The extracellular vesicles derived from spinach leaves were prepared in the same manner as in Example 4 above using commercially available spinach. The amount of sample added to each lane was 10 pg/lane in terms of protein content.

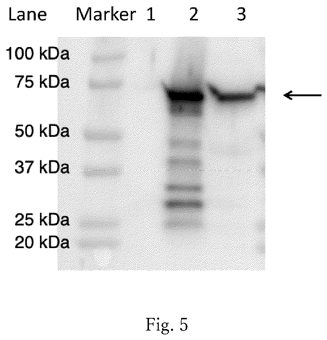

The results are shown in FIG. 5. FIG. 5 is an image showing the results of Western blotting on the EV sample derived from the red-fleshed dragon fruit. The "Marker" lane contains the marker, Lane 1 contains no sample, Lane 2 contains the EV sample, and Lane 3 contains the positive control (extracellular vesicles derived from spinach leaves). As shown in FIG. 5, an HSC70 band was detected in the EV sample derived from the red-fleshed dragon fruit, as well as in the positive control. This confirmed that the EV sample isolated in Example 1 above was composed of extracellular vesicles derived from a plant (red-fleshed dragon fruit).

Although the invention of the present application has been described above with reference to embodiments, the invention of the present application is not limited to these embodiments. Various modifications that can be understood by those skilled in the art can be made to the configurations and details of the invention of the present application without departing from the scope of the invention of the present application.

This application claims priority based on Japanese Patent Application No. 2022-135848, filed on August 29, 2022, the entire disclosure of which is hereby incorporated.

### Industrial Applicability

According to the present invention, for example, oxidation can be prevented, and the production of collagen, elastin, and the like can be promoted. Therefore, it is possible to improve conditions in living organisms associated with these proteins.

## Claims

1. An antioxidant comprising extracellular vesicles derived from a dragon fruit.

2. The antioxidant according to claim 1, wherein the extracellular vesicles are at least either one of extracellular vesicles derived from dragon fruit juice and extracellular vesicles derived from dragon fruit leaves.

3. The antioxidant according to claim 1 or 2, wherein a particle size distribution of the extracellular vesicles has a mean of 50 to 500 nm.

4. The antioxidant according to any one of claims 1 to 3, which is for parenteral use.

5. The antioxidant according to claim 4, which is for transdermal use.

6. The antioxidant according to any one of claims 1 to 3, which is for oral use.

7. The antioxidant according to any one of claims 1 to 6, wherein the dragon fruit is a red-fleshed dragon fruit.

8. A fibroblast promoter comprising extracellular vesicles derived from a dragon fruit.

9. The fibroblast promoter according to claim 8, wherein the extracellular vesicles are at least either one of extracellular vesicles derived from dragon fruit juice and extracellular vesicles derived from dragon fruit leaves.

10. The fibroblast promoter according to claim 9, wherein a particle size distribution of the extracellular vesicles has a mean of 50 to 500 nm.

11. The fibroblast promoter according to any one of claims 8 to 10, which is for parenteral use.

12. The fibroblast promoter according to claim 11, which is for transdermal use.

13. The fibroblast promoter according to any one of claims 8 to 10, which is for oral use.

14. The fibroblast promoter according to any one of claims 8 to 13, which promotes at least one of collagen production and elastin production in fibroblasts.

15. The fibroblast promoter according to any one of claims 8 to 14, wherein the dragon fruit is a red-fleshed dragon fruit.

16. An antioxidation method comprising adding extracellular vesicles derived from a dragon fruit to a subject.

17. A method for promoting collagen production in fibroblasts, the method comprising bringing extracellular vesicles derived from a dragon fruit into contact with fibroblasts.

18. A method for promoting elastin production in fibroblasts, the method comprising bringing extracellular vesicles derived from a dragon fruit into contact with fibroblasts.

19. A method for promoting elastin production in fibroblasts, the method comprising bringing extracellular vesicles derived from a dragon fruit into contact.
